# EUROPÄISCHE PATENTSCHRIFT

(11) **EP 2 529 662 B1**
(45) Veröffentlichungstag und Bekanntmachung des Hinweises auf die Patenterteilung: **30.04.2014**
(21) Anmeldenummer: 12162494.4
(22) Anmeldetag: 30.03.2012
(51) Int. Cl.: A61B 3/16, A61B 3/107

(54) **Ophthalmologisches Analyseverfahren und Analysesystem**
Ophthalmological analysis system and analysis method
Procédé d'analyse ophtalmologique et système d'analyse

(30) Priorität: 31.05.2011 DE 102011076793
(43) Veröffentlichungstag der Anmeldung: 05.12.2012
(73) Patentinhaber: Oculus Optikgeräte GmbH, 35582 Wetzlar-Dutenhofen (DE)
(72) Erfinder: Köst, Gert, 30159 Hannover (DE); Steinmüller, Andreas, 35435 Wettenberg (DE)
(74) Vertreter: advotec.

(56) Entgegenhaltungen:
- EP-A1- 2 397 068
- DE-U1-202005 002 562
- US-A- 6 120 444
- US-A1- 2005 030 473

## Beschreibung

Die Erfindung betrifft ein ophthalmologisches Analyseverfahren zur Messung eines intraokularen Drucks in einem Auge mit einem Analysesystem, sowie ein derartiges Analysesystem, gebildet aus einer Betätigungseinrichtung, mit der eine Hornhaut des Auges berührungsfrei verformt wird, wobei mit der Betätigungseinrichtung ein Luftstoß zur Verformung der Hornhaut auf das Auge aufgebracht wird, einem Beobachtungssystem, mit dem die Verformung der Hornhaut beobachtet und aufgenommen wird, wobei mit dem Beobachtungssystem Schnittbilder der unverformten und verformten Hornhaut aufgenommen werden, und einer Analyseeinrichtung, mit der aus den Schnittbildern der Hornhaut der intraokulare Druck abgeleitet wird.

Derartige Analyseverfahren und -systeme sind hinreichend bekannt und dienen primär einer möglichst genauen, berührungslosen Messung eines intraokularen Drucks in einem Auge. Dazu wird beispielsweise ein Non-Kontakt-Tonometer verwandt, mit Hilfe dessen ein Luftstoß auf das zu untersuchende Auge appliziert wird, wobei eine Stärke des Luftstoßes so gewählt wird, dass die Hornhaut des Auges unter Ausbildung einer konkaven Oberflächenform eingedrückt wird. Vor Erreichen eines Maximums einer Verformung der Hornhaut bzw. vor einem Einklappen der Hornhaut in Richtung der Augenlinse, bildet die Hornhaut kurzzeitig eine plane Fläche aus, die als sogenannter erster Applanationspunkt bezeichnet wird. Nach der maximalen Auslenkung der Hornhaut und einem Zurückklappen derselben in den ursprünglichen Zustand, wird ein ebensolcher zweiter Applanationspunkt von der Hornhaut durchlaufen. Durch eine Inbezugsetzung eines Drucks des Luftstoßes mit einem zeitlichen Verlauf der Applanation der Hornhaut ist es nun möglich, einen intraokularen Druck zu ermitteln. Die mit dem Non-Kontakt-Tonometer ermittelten Messwerte werden in Relation zu Vergleichsmesswerten gesetzt, welche mit einem relativ genauer messenden Applanationstonometer bzw. Kontakt-Tonometer ermittelt wurden, so dass als ein Ergebnis ein dem tatsächlichen intraokularen Druck angenäherter Augeninnendruck abgeleitet werden kann.

Jedoch ist ein mit einem Non-Kontakt-Tonometer gemessener intraokularer Druck gegenüber einer Druckmessung mit einem Applanationstonometer noch nicht hinreichend genau, da eine Messung unter anderem von der Hornhaut verfälscht wird. Um eine Messgenauigkeit zu verbessern, wurde daher versucht, die biomechanischen Eigenschaften einer Hornhaut während einer Non-Kontakt-Tonometermessung in diese mit einzubeziehen bzw. diese so bereits während der Messung zu ermitteln. Dazu wird ein Luftstoß auf eine Hornhaut aufgebracht, wobei ein Pumpendruck während des Verlaufs der Messung mittels eines Druckaufnehmers kontinuierlich erfasst wird. Weiter wird ein zeitlicher Verlauf der Messung erfasst sowie ein erster und ein zweiter Applanationspunkt der Hornhaut optisch detektiert. Ein intraokularer Druck kann nun beispielsweise durch eine Bestimmung der zum Zeitpunkt der ersten und zweiten Applanation herrschenden Drücke abgeleitet werden, insbesondere da die zur Biegung der Hornhaut notwendigen Kräfte beim Ein- bzw. Ausklappen der Hornhaut als gleich groß angenommen werden und sich somit gegenseitig aufheben. Folglich resultiert ein intraokularer Druck aus einem Mittelwert der zum Einklappen und Ausklappen der Hornhaut aufgewendeten Kraft, aufgebracht durch den Luftstoß.

Alternativ ist es bekannt, eine Hysterese zwischen dem ersten und dem zweitem Applanationspunkt zu bestimmen und auf Basis der Hysteresemessung den intraokularen Druck abzuleiten bzw. zu korrigieren. Nachteilig bei diesen Messverfahren ist, dass eine durch einen Luftstoß bewirkte Bewegung der Hornhaut dynamischen Effekten unterliegt, welche derartige Zeit-/Druckmessungen verfälschen können, insbesondere da die dynamischen Effekte bei den beschriebenen Non-Kontakt-Tonometermessungen nicht berücksichtigt werden können.

Insgesamt sind daher die aus dem Stand der Technik bekannten Analyseverfahren und -systeme mit voneinander abhängiger, paralleler Druck- und Zeitmessung bei gleichzeitiger Detektion der Applanationspunkte gegenüber einer Messung mit einem Kontakt-Tonometer noch vergleichsweise ungenau. Selbst wenn man die vorgenannten, möglichen Fehlerquellen für Ungenauigkeiten außer Betracht lässt, treten bei derartigen Non-Kontakt-Tonometermessungen offensichtlich noch weitere, eine Messung verfälschende Effekte auf.

Aus der DE 20 2005 002 562 U1 ist ein Non-Kontakt-Tonometer bekannt, mit dem eine Hornhaut eines Auges mittels eines Luftstoßes berührungsfrei verformt werden kann. Weiter umfasst das Non-Kontakt-Tonometer ein Beobachtungssystem, mit dem die Verformung der Hornhaut mittels einer Scheimpflugkamera aufgenommen werden kann. So können Schnittbilder der unverformten und verformten Hornhaut aufgenommen werden, wobei aus den Schnittbildern mittels einer Analyseeinrichtung der intraokulare Druck abgeleitet wird.

Aus der EP 2 397 068 A1 ist ebenfalls ein Non-Kontakt-Tonometer mit einer Scheimpflugkamera zur Aufnahme von Schnittbildern bekannt. Hier ist eine Ableitung des intraokularen Drucks unter Berücksichtigung einer Materialeigenschaft der Hornhaut vorgesehen. Die Materialeigenschaft ergibt sich aus der Größe einer ersten Applanationsfläche im Vergleich zu weiteren Verformungsflächen der Hornhaut.

Die US 2005/0030473 A1 betrifft ein Non-Kontakt-Tonometer, bei dem eine Messung eines Augeninnendrucks unter anderem durch Einbeziehung einer Steifigkeit einer Hornhaut korrigiert wird.

Der vorliegenden Erfindung liegt daher die Aufgabe zugrunde, ein ophthalmologisches Analyseverfahren zur Messung eines intraokularen Drucks in einem Auge sowie ein derartiges Analysesystem vorzuschlagen, mit dem eine vergleichsweise verbesserte Messgenauigkeit erzielbar ist.

Diese Aufgabe wird durch ein ophthalmologisches Analyseverfahren mit den Merkmalen des Anspruchs 1 und ein Analysesystem mit den Merkmalen des Anspruchs 14 gelöst.

Bei dem erfindungsgemäßen, ophthalmologischen Analyseverfahren zur Messung eines intraokularen Drucks in einem Auge mit einem Analysesystem, umfasst das Analysensystem eine Betätigungseinrichtung, mit der eine Hornhaut des Auges berührungsfrei verformt wird, wobei mit der Betätigungseinrichtung ein Luftstoß zur Verformung der Hornhaut auf das Auge aufgebracht wird, ein Beobachtungssystem, mit dem die Verformung der Hornhaut beobachtet und aufgenommen wird, wobei mit dem Beobachtungssystem Schnittbilder der unverformten und verformten Hornhaut aufgenommen werden, und eine Analyseeinrichtung, mit der aus den Schnittbildern der Hornhaut der intraokulare Druck abgeleitet wird, wobei die aufgenommenen Schnittbilder der verformten Hornhaut relativ zu einem aufgenommenen Schnittbild der unverformten Hornhaut korrigiert werden, wobei jeweils die Schnittbilder der verformten Hornhaut relativ zu dem Schnittbild der unverformten Hornhaut um einen räumlichen, parallelen Versatz in Richtung einer Sehachse des Auges oder einer Geräteachse des Analysesystems korrigiert werden, wobei der intraokulare Druck unter Berücksichtigung der Korrektur abgeleitet wird.

Überraschenderweise wurde bei einer Beobachtung eines Gesamtsystems des Auges während einer Non-Kontakt-Tonometermessung festgestellt, dass zwischen einem aufgenommenen Schnittbild der unverformten Hornhaut und Schnittbildern der verformten Hornhaut Unterschiede hinsichtlich einer Relativposition bestehen. So wird durch das Aufbringen des Luftstoßes auf die Hornhaut des Auges eine Kraft aufgebracht, die eine Bewegung des gesamten Auges bewirkt. Da bei den aus dem Stand der Technik bekannten Non-Kontakt-Tonometermessungen lediglich ein vorderer Bereich des Auges beobachtet wird, kann eine Bewegung des Gesamtsystems des Auges nicht bei der Ableitung des intraokularen Drucks berücksichtigt werden, was zu einer Verfälschung der ermittelten Messwerte führt. Nach dem erfindungsgemäßen Analyseverfahren ist es daher vorgesehen die aufgenommenen Schnittbilder der verformten Hornhaut, welche durch eine Bewegung des Gesamtsystems des Auges relativ zu einem Schnittbild der unverformten Hornhaut verfälscht sind, um den betreffenden Fehler zu korrigieren und erst dann den intraokularen Druck aus den aufgenommenen Schnittbildern der verformten und unverformten Hornhaut abzuleiten. So kann wirkungsvoll eine bisher nicht berücksichtigte Fehlerquelle bei einer Messung des intraokularen Drucks mittels eines Non-Kontakt-Tonometers ausgeschlossen werden, und eine wesentlich verbesserte Messgenauigkeit erzielt wird.

Erfindungsgemäß erfolgt eine Korrektur der Schnittbilder der verformten Hornhaut dadurch, dass jeweils die Schnittbilder der verformten Hornhaut relativ zu dem Schnittbild der unverformten Hornhaut um einen räumlichen Versatz korrigiert werden. Eine durch den Luftstoß hervorgerufene Bewegung des Gesamtsystems des Auges in einer der Betätigungseinrichtung abgewandten Richtung bewirkt folglich einen räumlichen, parallelen Versatz des gesamten Auges in Richtung einer Sehachse bzw. Geräteachse des Analysesystems. Der dadurch auftretende Fehler bei einer Messung kann durch eine Ermittlung des Versatzes besonders einfach korrigiert werden. Es ist dann lediglich notwendig die Schnittbilder der verformten Hornhaut jeweils um den räumlichen Versatz relativ zu den Schnittbildern der unverformten Hornhaut zu korrigieren.

So kann vorgesehen sein, dass das Schnittbild der unverformten Hornhaut als ein Bezugspunkt für die Schnittbilder der verformten Hornhaut verwendet wird. Folglich kann eine räumliche Abweichung der Schnittbilder der verformten Hornhaut, bedingt durch eine Bewegung des Gesamtsystems des Auges, vom Bezugspunkt bzw. von zumindest einem Schnittbild der unverformten Hornhaut korrigiert werden. Das Schnittbild der unverformten Hornhaut bzw. eine daraus zu entnehmende Position der unverformten Hornhaut dient demnach als ein Referenzpunkt für die räumliche Abweichung der verformten Hornhaut sowie des Gesamtsystems des Auges.

Eine derartige Korrektur kann noch weiter verbessert werden, wenn eine Funktion des Versatzes berücksichtigt wird. Das heißt eine Bewegung des gesamten Auges relativ zu einem Zeitraum einer Verformung der Hornhaut muss nicht zwangsläufig parallel und linear zu der Verformung der Hornhaut verlaufen. So kann beispielsweise bei einer Korrektur berücksichtigt werden, dass eine Bewegung des gesamten Auges relativ zu einer Bewegung der Hornhaut aufgrund der Unterschiede der jeweiligen Massen verzögert beginnt und auch bei Erreichen eines Verformungsmaximums der Hornhaut noch nicht ein Maximum eines möglichen Versatzes erreicht hat. Weiter kann berücksichtigt werden, dass die Bewegung des Gesamtsystems des Auges relativ zu einem Verformungszeitraum der Hornhaut nicht linear verläuft, da nach dem Luftstoß auf das Auge bereits eine das Auge aufnehmende Augenhöhle der Bewegung des Auges einen Widerstand entgegensetzt.

Auch kann ein Zeitabschnitt zwischen Beginn und Ende der Verformung der Hornhaut gemessen werden. So können insbesondere alle aufgenommenen Schnittbilder jeweils einem bestimmten Zeitpunkt der Messung zugeordnet werden, wodurch ein zeitlicher Ablauf der Verformung nachvollziehbar wird. Insbesondere kann ein Zeitpunkt der ersten und der zweiten Applanation der Hornhaut und somit ein zeitlicher Abstand genau bestimmt werden. So kann auch die Ermittlung dieses Zeitabschnittes zur Bestimmung des betreffenden Korrekturwertes verwendet werden. Weiter kann zur Ableitung des Korrekturwertes ein Zeitabschnitt der gesamten Verformung der Hornhaut herangezogen werden.

Auch kann eine Geschwindigkeit der bewegten Hornhaut gemessen werden. Wenn insbesondere der Zeitverlauf einer Verformung der Hornhaut bekannt ist, kann so auch eine Dynamik der Verformung oder des Versatzes untersucht werden, um besondere dynamische Effekte bei der Verformung in Hinblick auf die notwendige Korrektur zu bewerten. Beispielsweise kann sich ein Nachschwingen der Hornhaut bei einem Luftstoß somit nicht mehr verfälschend auf ein Messergebnis auswirken, wenn das Nachschwingen bei der Messung berücksichtigt wird. Auch wird so eine Geschwindigkeit eines Luftstoßes in Bezug auf sonst unerwünschte dynamische Effekte für eine Messung beliebig wählbar. Auch ist es möglich von der gemessenen Geschwindigkeit auf eine Eindrücktiefe bzw. maximale Amplitude der Verformung und des Versatzes zu schließen, da zwischen diesen Größen ein funktionaler Zusammenhang besteht.

Eine besonders genaue Korrektur wird möglich, wenn ein Versatz eines Augapfels gemessen wird. So kann eine Bewegung des Gesamtsystems des Auges vollständig erfasst und damit korrigiert werden.

Auch ist es möglich, einen Versatz eines Augenhintergrundes zu messen. Beispielsweise kann mittels eines Interferometers, oder einer anderen, geeigneten Messvorrichtung, eine Augenlänge oder ein Abstand eines Augenhintergrundes bzw. eines Punktes einer Netzhaut des Auges relativ zur Messvorrichtung bestimmt werden. Während der Verformung der Hornhaut mittels des Luftstoßes kann dieser Abstand dann kontinuierlich gemessen werden, wodurch ein Versatz der Netzhaut infolge des Luftstoßes ermittelt werden kann. So kann im Wesentlichen eine Bewegung des Gesamtsystems des Auges relativ genau gemessen werden.

Dazu ist es weiter möglich, alleine oder zusätzlich zu den vorbeschriebenen Verfahrensabwandlungen, einen Versatz aus den Schnittbildern der verformten und der unverformten Hornhaut abzuleiten. Wenn eine Serie von Schnittbildern der verformten Hornhaut aufgenommen wird, kann anhand eines Verformungsverlaufs, welcher sich aus den Schnittbildern ergibt, festgestellt werden, ob ein Versatz des Auges infolge des Luftstoßes erfolgt, und wie groß dieser Versatz ist.

Dieser Versatz kann aus einer Mehrzahl von Referenzpunkten in einem einer Sehachse oder Geräteachse abgewandten Randbereich der Schnittbilder ermittelt werden. In einem Randbereich einer Schnittbildaufnahme, beispielsweise in einem Übergangsbereich zu einer Lederhaut des Auges, erfolgt eine wesentlich geringere Verformung der Hornhaut infolge des Luftstoßes. Vielmehr kann sich hier eine aus einem Vergleich der Schnittbilder ergebende Verformung durch einen Versatz des betreffenden Hornhautbereiches infolge einer Bewegung des Gesamtsystems des Auges ergeben. Wenn ein etwaiger Einfluss bzw. Anteil eines Versatzes an einer Verformung des Randbereichs der Hornhaut bekannt ist, kann dieser zur Korrektur der Schnittbilder der verformten Hornhaut berücksichtigt werden.

Weiter ist es vorteilhaft, wenn ein maximaler Versatz ermittelt wird. So kann leicht festgestellt werden, zu welchem Zeitpunkt der Verformung der Hornhaut ein maximaler Versatz des Gesamtsystems des Auges erreicht wird. Auch können diese Messwerte zu einer noch genaueren Korrektur der aufgenommenen Schnittbilder der verformten Hornhaut herangezogen werden.

Weiter ist zu beachten, dass bei dem erfindungsgemäßen Verfahren eine Druckmessung eines Pumpendrucks nicht notwendig ist. So kann jede beliebige Messung eines intraokularen Drucks immer mit dem gleichen, konstanten Pumpendruck durchgeführt werden. Da hier keine Variation einer Höhe des Pumpendrucks bzw. eine Zeitsynchronisation des Pumpendrucks erfolgen muss, können eine Reihe von möglichen Fehlerquellen ausgeschlossen und eine besonders genaue Messung durchgeführt werden.

Weiter ist es vorteilhaft, wenn ein Pumpendruck zur Erzeugung des Luftstoßes relativ zu einer Zeitdauer desselben in Form einer Glockenkurve verläuft. So kann der Pumpendruck für jede individuelle Messung identisch und vollkommen unbeeinflusst in Form des Luftstoßes auf die Hornhaut einwirken. Die Glockenkurve kann dabei unter anderem eine symmetrische Gestalt aufweisen.

Auch kann ein maximaler Pumpendruck zur Erzeugung des Luftstoßes bei vorangehenden und nachfolgenden Messungen gleich sein. So kann eine besonders gute Vergleichbarkeit von verschiedenen Messungen ermöglicht werden. Der maximale Pumpendruck kann beispielsweise 70 mm Hg betragen.

Um einen Pumpendruck dennoch gegebenenfalls korrigieren zu können bzw. um einen gewünschten Druckverlauf zu überprüfen, kann ein Pumpendruck zur Erzeugung des Luftstoßes bei Erreichen eines Applanationspunktes der Hornhaut gemessen werden. Beispielsweise kann eine Pumpe über einen Drucksensor verfügen, der die Überwachung des Pumpendrucks während der gesamten Messung ermöglicht. Dadurch können mögliche Fehler hinsichtlich des Pumpendrucks bei der Messung ausgeschlossen und eine Kontinuität aufeinanderfolgender Messungen sichergestellt werden.

Um einen Korrekturwert noch genauer zu bestimmen, kann eine maximale Verformung der Hornhaut aus den Schnittbildern der Hornhaut abgeleitet werden. Folglich kann eine maximale Eindrücktiefe der Hornhaut aus den Schnittbildern der Hornhaut ermittelt werden, wobei ergänzend auch ein Zeitpunkt der maximalen Verformung der Hornhaut zumindest relativ zu einem der Applanationspunkte festgestellt werden kann.

Die notwendige Korrektur der Schnittbilder der Hornhaut kann noch genauer bestimmt werden, wenn eine Amplitude der Verformung der Hornhaut aus den Schnittbildern der Hornhaut abgeleitet wird. So kann der genaue geometrische Verlauf der Verformung und des Versatzes leicht nachvollzogen werden. Das heißt, es kann zu jedem Zeitpunkt der Verformung die zu diesem Zeitpunkt existente, geometrische Gestalt der Verformung aufgenommen werden, so dass der geometrische Verlauf der Verformung in Art eines Films der Verformung erfasst werden kann. Beispielsweise ist auch ein Nachschwingen der Hornhaut nach einem Ausklappen bzw. einen zweiten Applanationspunkt so gut erfassbar.

Optional kann auch bei Erreichen eines Applanationspunktes der Hornhaut eine Größe einer ebenen Applanationsfläche gemessen werden. Beispielsweise kann eine Größe der Applanationsfläche bzw. deren Durchmesser und/oder deren Form als ein Indikator für ein Wegpunkt der Verformung der Hornhaut berücksichtigt werden.

Weiter kann vorgesehen sein, dass die Verformungsfläche bzw. die Applanationsfläche in einem bestimmten Zeitabschnitt der Verformung relativ zu einem anderen, messbaren Punkt oder einem Versatz der Hornhaut während der Verformung zur Definition des Versatzes der Hornhaut bzw. der Schnittbilder infolge einer Augenbewegung herangezogen wird. Weiter können die ermittelte Abweichung und die Relativwerte der betreffenden Position in einer Datenbank gespeichert und verglichen werden. So kann für die in der Datenbank gespeicherten Werte ein objektiver Augeninnendruck oder auch ein entsprechender Korrekturwert bekannt sein, so dass der objektive intraokulare Druck des gemessenen Auges unter Berücksichtigung des Versatzes der Hornhaut bzw. des gesamten Auges abgeleitet werden kann.

Der Versatz der Schnittbilder der Hornhaut kann noch weiter differenziert werden, wenn die Verformung der Hornhaut durch eine freie Schwingung der Hornhaut fortgesetzt wird, und wenn eine weitere Korrektur der freien Schwingung der Hornhaut erfolgt. Folglich kann vorgesehen sein, mittels des Beobachtungssystems Schnittbilder der Hornhaut über die eigentliche Verformung der Hornhaut hinaus aufzunehmen, um ein Ausschwingen bzw. die freie Schwingung der Hornhaut zu ermitteln.

In einer vorteilhaften Ausführungsform des Analyseverfahrens kann das Beobachtungssystem eine Kamera und eine Beleuchtungseinrichtung in einer Scheimpfluganordnung umfassen, wobei dann mittels der Kamera die Schnittbilder aufgenommen werden können. Das heißt die Kamera kann relativ zu einer optischen Achse einer Spaltbeleuchtungseinrichtung zur Beleuchtung des Auges in einer Scheimpfluganordnung angeordnet sein, so dass ein beleuchtetes Querschnittsbild des Auges mit der Kamera aufgenommen werden kann. Eine Kamera kann beispielsweise auch als eine Hochgeschwindigkeitskamera verwendet werden, die mindestens 4000 Bilder pro Sekunde aufnehmen kann. Auch kann die optische Achse der Spaltbeleuchtungseinrichtung in eine Sehachse des Auges fallen bzw. mit dieser übereinstimmen. Vorzugsweise kann dann auch eine Wirkrichtung des Luftstoßes koaxial zur optischen Achse der Spaltbeleuchtungseinrichtung verlaufen.

Das erfindungsgemäße ophthalmologische Analysesystem zur Messung eines intraokularen Drucks in einem Auge umfasst eine Betätigungseinrichtung, mit der eine Hornhaut des Auges berührungsfrei verformt werden kann, wobei mit der Betätigungseinrichtung ein Luftstoß zur Verformung der Hornhaut auf das Auge aufgebracht werden kann, ein Beobachtungssystem, mit dem die Verformung der Hornhaut beobachtet und aufgenommen werden kann, wobei mit dem Beobachtungssystem Schnittbilder der unverformten und verformten Hornhaut aufgenommen werden können, und eine Analyseeinrichtung, mit der aus den Schnittbildern der Hornhaut der intraokulare Druck abgeleitet werden kann, wobei die aufgenommenen Schnittbilder der verformten Hornhaut relativ zu einem aufgenommenen Schnittbild der unverformten Hornhaut korrigiert werden, wobei jeweils die Schnittbilder der verformten Hornhaut relativ zu dem Schnittbild der unverformten Hornhaut um einen räumlichen, parallelen Versatz in Richtung einer Sehachse des Auges oder einer Geräteachse des Analysesystems korrigiert werden, wobei der intraokulare Druck unter Berücksichtigung der Korrektur abgeleitet wird.

Weitere vorteilhafte Ausführungsformen des Analysesystems ergeben sich aus den Merkmalsbeschreibungen der auf den Verfahrensanspruch 1 rückbezogenen Unteransprüche.

Nachfolgend wird eine bevorzugte Ausführungsform der Erfindung unter Bezugnahme auf die beigefügten Zeichnungen näher erläutert.

Es zeigen:
- **Fig. 1a bis 1c:**: eine Verformung einer Hornhaut eines Auges während einer Messung in einer Längsschnittansicht.
- **Fig. 2:**: eine Diagrammdarstellung von Pumpendruck und -zeit während einer Messung.

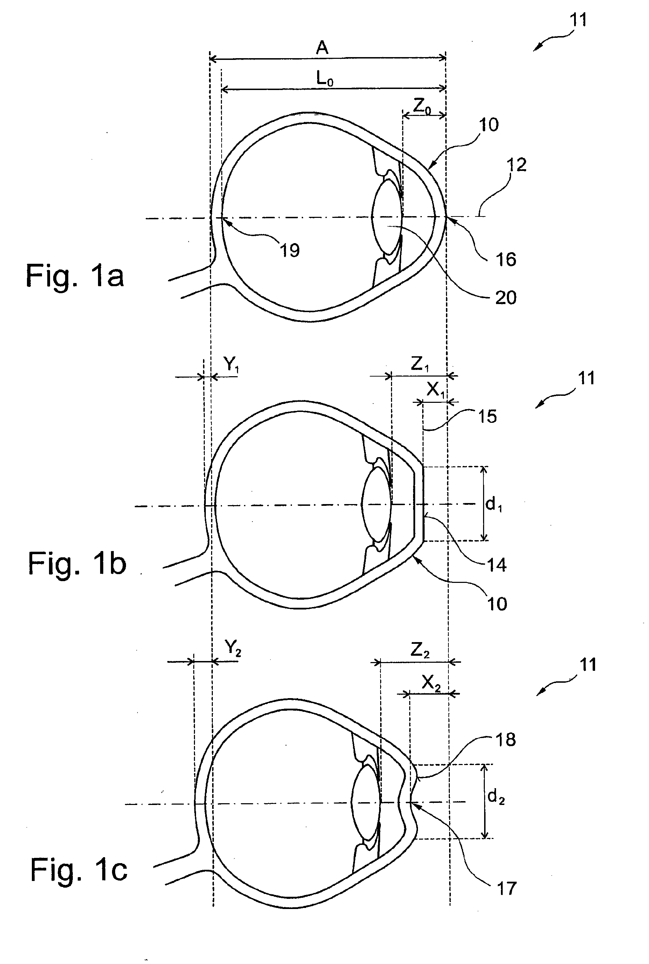

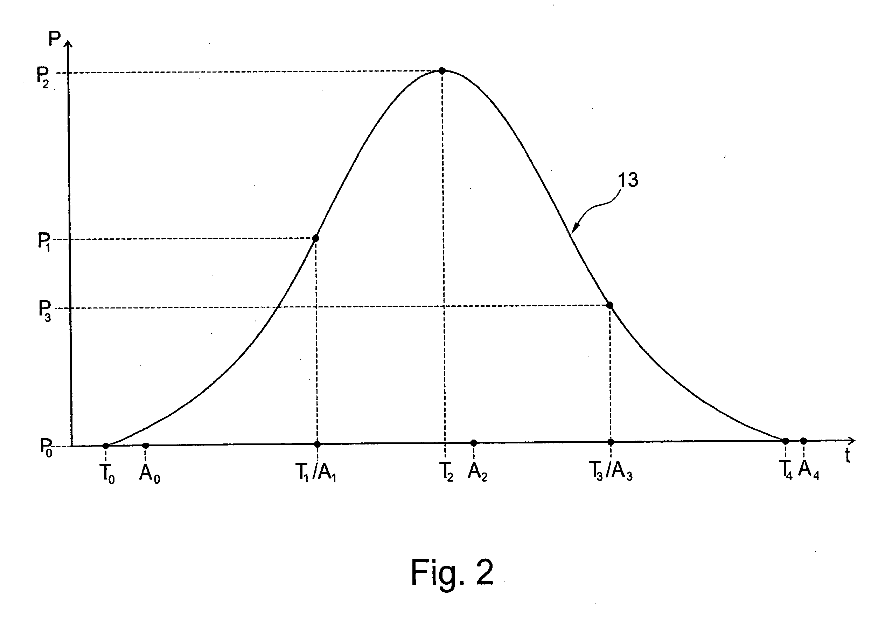

Die **Fig. 1a** bis **1c** zeigen ausgewählte Verformungszustände einer Hornhaut 10 eines Auges 11 während einer einzelnen Messung eines Augeninnendrucks durch ein hier nicht dargestelltes Analysesystem. Die Darstellungen sind jeweils Längsschnittdarstellungen entlang einer Sehachse 12 des Auges 11. **Fig. 2** ist eine Diagrammdarstellung mit einer Zeit t auf der Abzissenachse und einem Pumpendruck p auf der Ordinatenachse. Der Pumpendruck verläuft unabhängig vom Einsatz eines hier nicht dargestellten Beobachtungssystems bzw. einer Scheimpflugkamera mit einer Spaltbeleuchtungseinrichtung in Art einer symmetrischen Glockenkurve 13, beginnend mit einem Druck P₀ bei einem Startpunkt To der Pumpe bis hin zu einem maximalen Pumpendruck P₂ zum Zeitpunkt T₂ und wieder abfallend bis hin zu dem Pumpendruck P₀ in einem Endpunkt T₄. Der durch den Start der Pumpe bei T₀ abgegebene Luftstoß auf die Hornhaut 10 führt zu einer ersten, mittels des Beobachtungssystems aufnehmbaren Verformung der Hornhaut 10 unmittelbar nach dem Zeitpunkt A₀. **Fig. 1a** repräsentiert die Gestalt der noch nicht verformten Hornhaut 10 zum Zeitpunkt A₀. Mit steigendem Pumpendruck kommt es zum Zeitpunkt A₁ zu einer vollständigen Applanation der Hornhaut 10 gemäß **Fig. 1b****,** wobei wie hier dargestellt, eine Applanationsfläche 14 mit einem Durchmesser d₁ ausgebildet wird, die im Wesentlichen eben ist und in einer Applanationsebene 15 liegt. Die Hornhaut ist dann um ein Maß X₁ vom Apex 16 der Hornhaut 10 entfernt bzw. eingedrückt. Optional und nicht notwendigerweise kann bei diesem sogenannten ersten Applanationspunkt zum Zeitpunkt A₁ ein Pumpendruck P₁ zum übereinstimmenden Zeitpunkt T₁ ermittelt werden. Nach Erreichen des Pumpendrucks P₂ kommt es zu einer maximalen Verformung der Hornhaut 10 zum Zeitpunkt A₂ entsprechend der Darstellung in **Fig. 1c****.** Ein eine maximale Verformung bestimmender Punkt 17 ist dabei um ein Maß X₂ vom Apex 16 der Hornhaut 10 entfernt. Es handelt sich in diesem Fall also um eine maximale Auslenkung einer Amplitude der Verformung. Bei dieser maximalen Amplitude der Verformung wird ein Durchmesser d₂ einer konkaven Verformungsfläche 18 ausgebildet und erfasst. Der Durchmesser d₂ ist durch einen Abstand von zwei gegenüberliegenden Punkten einer Längsschnittebene der Hornhaut 10 definiert, wobei die Punkte jeweils die dem Analysesystem am nächsten zugewandten Punkte der Hornhaut 10 repräsentieren. Nachfolgend kommt es zu einer Rückbewegung bzw. einem Ausschwingen der Hornhaut 10, wobei zum Zeitpunkt A₃ der sogenannte zweite Applanationspunkt erreicht wird, der hier nicht weiter dargestellt ist. Auch ist es optional möglich einen Pumpendruck P₃ zum übereinstimmenden Zeitpunkt T₃ zu bestimmen. Nach dem Rückgang des Pumpendrucks auf den Ursprungswert Po zum Zeitpunkt T₄ gelangt die Hornhaut 10 zum Zeitpunkt A₄ wieder in ihre in **Fig. 1a** dargestellte Ausgangslage. Entsprechend der vorangehenden Beschreibung einer einzelnen Messung eines intraokularen Drucks eines Auges werden die beschriebenen Verformungszustände der Hornhaut 10, welche durch die jeweiligen mit A₀ bis A₄ gekennzeichneten Zeitpunkte charakterisiert sind, ermittelt. Dabei werden insbesondere unabhängig von einem Pumpendruck p Zeitabstände der betreffenden Zeitpunkte A₀ bis A₄ sowie die Maße bzw. Eindrücktiefen X₁ und X₂ erfasst. Wie weiter den **Fig. 1a** bis **1c** zu entnehmen ist, weist das Auge 11 eine Augenlänge A und einen Abstand vom Apex 16 zu einer Netzhaut 19 entlang der Sehachse 12 mit einer Länge L₀ auf. Weiter ist eine Länge Z₀ vom Apex 16 bis zu einer Linse 20 messbar. Eine Messung der Länge Z₀ kann beispielsweise mittels einer Kamera in einer Scheimpfluganordnung und eine Länge L₀ mit einem Interferometer gemessen werden. Bei der Verformung der Hornhaut 10 mittels des Luftstoßes, wie in **Fig. 1b** gezeigt, erfolgt ein Versatz des gesamten Auges 11 in einer hier nicht dargestellten Augenhöhle entlang der Sehachse 12 um die Länge Y₁. Da die Hornhaut 10 messbar um die Eindrücktiefe X₁ verformt wird, ergibt sich eine tatsächliche Verformung der Hornhaut 10 relativ zum Apex 16 entsprechend der Gleichung Xₖₒᵣᵣₑₖₜᵤᵣ = X₁ - Y₁. Folglich wird das in **Fig. 1b** gezeigte Schnittbild um die Länge Y₁ korrigiert, um das korrigierte Schnittbild zur Ableitung des intraokularen Drucks zu verwenden. Bei der weitergehenden Verformung der Hornhaut 10 bis hin zur Eindrücktiefe X₂ erfolgt ein ebenfalls weitergehender Versatz des Auges 11 um die Länge Y₂. Das in **Fig. 1c** gezeigte Schnittbild des verformten Auges 11 wird dann, wie zuvor beschrieben, um die Länge Y₂ entlang der Sehachse 12 verschoben bzw. korrigiert. Zusätzlich oder alternativ kann vorgesehen sein, eine Korrektur der betreffenden Schnittbilder anhand der Differenzen der Längen Z₀, Z₁ und Z₂ analog durchzuführen.

Durch eine Verwendung derart korrigierter Schnittbilder des verformten Auges 11 bzw. der Hornhaut 10 wird es möglich, eine wesentliche Fehlerquelle bei der Ableitung des intraokularen Drucks aus den Schnittbildern der Hornhaut auszuschließen und so ein gegenüber den aus dem Stand der Technik bekannten Messverfahren genaueres Messergebnis eines intraokularen Drucks zu erhalten.

## Patentansprüche

1. Ophthalmologisches Analyseverfahren zur Messung eines intraokularen Drucks in einem Auge (11) mit einem Analysesystem, gebildet aus einer Betätigungseinrichtung, mit der eine Hornhaut (10) des Auges berührungsfrei verformt wird, wobei mit der Betätigungseinrichtung ein Luftstoß zur Verformung der Hornhaut auf das Auge aufgebracht wird, einem Beobachtungssystem, mit dem die Verformung der Hornhaut beobachtet und aufgenommen wird, wobei mit dem Beobachtungssystem Schnittbilder der unverformten und verformten Hornhaut aufgenommen werden, und einer Analyseeinrichtung, mit der aus den Schnittbildern der Hornhaut der intraokulare Druck abgeleitet wird,
**dadurch gekennzeichnet,**
**dass** die aufgenommenen Schnittbilder der verformten Hornhaut relativ zu einem aufgenommenen Schnittbild der unverformten Hornhaut korrigiert werden, wobei jeweils die Schnittbilder der verformten Hornhaut relativ zu dem Schnittbild der unverformten Hornhaut um einen räumlichen, parallelen Versatz in Richtung einer Sehachse (12) des Auges oder einer Geräteachse des Analysesystems korrigiert werden, wobei der intraokulare Druck unter Berücksichtigung der Korrektur abgeleitet wird.

2. Analyseverfahren nach Anspruch 1,
**dadurch gekennzeichnet,**
**dass** das Schnittbild der unverformten Hornhaut (10) als ein Bezugspunkt für die Schnittbilder der verformten Hornhaut verwendet wird.

3. Analyseverfahren nach Anspruch 1 oder 2,
**dadurch gekennzeichnet,**
**dass** eine Funktion des Versatzes berücksichtigt wird.

4. Analyseverfahren nach einem der vorangehenden Ansprüche,
**dadurch gekennzeichnet,**
**dass** ein Versatz eines Augapfels gemessen wird.

5. Analyseverfahren nach einem der vorangehenden Ansprüche,
**dadurch gekennzeichnet,**
**dass** ein Versatz eines Augenhintergrundes (19) gemessen wird.

6. Analyseverfahren nach einem der vorangehenden Ansprüche,
**dadurch gekennzeichnet,**
**dass** ein Versatz aus den Schnittbildern der verformten und der unverformten Hornhaut (10) abgeleitet wird.

7. Analyseverfahren nach Anspruch 6,
**dadurch gekennzeichnet,**
**dass** der Versatz aus einer Mehrzahl von Referenzpunkten in einem der Sehachse (12) oder der Geräteachse abgewandten Randbereich der Schnittbilder ermittelt wird.

8. Analyseverfahren nach einem der vorangehenden Ansprüche,
**dadurch gekennzeichnet,**
**dass** ein maximaler Versatz ermittelt wird.

9. Analyseverfahren nach einem der vorangehenden Ansprüche,
**dadurch gekennzeichnet,**
**dass** ein Pumpendruck zur Erzeugung des Luftstoßes relativ zu einer Zeitdauer desselben in Form einer Glockenkurve (13) verläuft.

10. Analyseverfahren nach Anspruch 9,
**dadurch gekennzeichnet,**
**dass** ein maximaler Pumpendruck zur Erzeugung des Luftstoßes bei vorangehenden und nachfolgenden Messungen gleich ist.

11. Analyseverfahren nach Anspruch 9 oder 10,
**dadurch gekennzeichnet,**
**dass** ein Pumpendruck zur Erzeugung des Luftstoßes bei Erreichen eines Applanationspunktes der Hornhaut (10) gemessen wird.

12. Analyseverfahren nach einem der vorangehenden Ansprüche,
**dadurch gekennzeichnet,**
**dass** eine maximale Verformung der Hornhaut (10) aus den Schnittbildern der Hornhaut abgeleitet wird.

13. Analyseverfahren nach einem der vorangehenden Ansprüche,
**dadurch gekennzeichnet,**
**dass** das Beobachtungssystem eine Kamera und eine Beleuchtungseinrichtung in einer Scheimpfluganordnung umfasst, wobei mittels der Kamera die Schnittbilder aufgenommen werden.

14. Ophthalmologisches Analysesystem zur Messung eines intraokularen Drucks in einem Auge (11), umfassend eine Betätigungseinrichtung, mit der eine Hornhaut (10) des Auges berührungsfrei verformt werden kann, wobei mit der Betätigungseinrichtung ein Luftstoß zur Verformung der Hornhaut auf das Auge aufgebracht werden kann, ein Beobachtungssystem, mit dem die Verformung der Hornhaut beobachtet und aufgenommen werden kann, wobei mit dem Beobachtungssystem Schnittbilder der unverformten und verformten Hornhaut aufgenommen werden können, und eine Analyseeinrichtung, mit der aus den Schnittbildern der Hornhaut der intraokulare Druck abgeleitet werden kann,
**dadurch gekennzeichnet,**
**dass** die aufgenommenen Schnittbilder der verformten Hornhaut relativ zu einem aufgenommenen Schnittbild der unverformten Hornhaut korrigiert werden, wobei jeweils die Schnittbilder der verformten Hornhaut relativ zu dem Schnittbild der unverformten Hornhaut um einen räumlichen, parallelen Versatz in Richtung einer Sehachse (12) des Auges oder einer Geräteachse des Analysesystems korrigiert werden, wobei der intraokulare Druck unter Berücksichtigung der Korrektur abgeleitet wird.

## Claims

1. An ophthalmological analysis method for measuring an intraocular pressure in an eye (11) using an analysis system formed from an actuation device, with which a cornea (10) of the eye is deformed in a contact-free manner, a puff of air being applied to the eye using the actuation device to deform the cornea, formed from a monitoring system, with which the deformation of the cornea is monitored and recorded, sectional images of the undeformed and deformed cornea being recorded using the monitoring system, and formed from an analysis device, with which the intraocular pressure is derived from the sectional images of the cornea,
**characterised in that**
the recorded sectional images of the deformed cornea are corrected relative to a recorded sectional image of the undeformed cornea, the sectional images of the deformed cornea each being corrected by a spatial parallel offset in the direction of an optical axis (12) of the eye or of a device axis of the analysis system, relative to the sectional image of the undeformed cornea, the intraocular pressure being derived taking the correction into account.

2. The analysis method according to claim 1,
**characterised in that**
the sectional image of the undeformed cornea (10) is used as a reference point for the sectional images of the deformed cornea.

3. The analysis method according to claim 1 or 2,
**characterised in that**
a function of the offset is taken into account.

4. The analysis method according to any one of the preceding claims, **characterised in that**
an offset of an eyeball is measured.

5. The analysis method according to any one of the preceding claims, **characterised in that**
an offset of an ocular fundus (19) is measured.

6. The analysis method according to any one of the preceding claims, **characterised in that**
an offset is derived from the sectional images of the deformed and of the undeformed cornea (10).

7. The analysis method according to claim 6,
**characterised in that**
the offset is established from a plurality of reference points in an edge region of the sectional images remote from the optical axis (12) or from the device axis.

8. The analysis method according to any one of the preceding claims, **characterised in that**
a maximum offset is established.

9. The analysis method according to any one of the preceding claims, **characterised in that**
a pump pressure for producing the puff of air progresses in the form of a bell curve (13) relative to a duration thereof.

10. The analysis method according to claim 9,
**characterised in that**
a maximum pump pressure for producing the puff of air is identical in previous and subsequent measurements.

11. The analysis method according to claim 9 or 10,
**characterised in that**
a pump pressure for producing the puff of air is measured once an applanation point of the cornea (10) has been reached.

12. The analysis method according to any one of the preceding claims, **characterised in that**
a maximum deformation of the cornea (10) is derived from the sectional images of the cornea.

13. The analysis method according to any one of the preceding claims, **characterised in that**
the monitoring system comprises a camera and an illumination device in a Scheimpflug arrangement, the sectional images being recorded by means of the camera.

14. An ophthalmological analysis system for measuring an intraocular pressure in an eye (11), comprising an actuation device, with which a cornea (10) of the eye can be deformed in a contact-free manner, it being possible to apply a puff of air to the eye using the actuation device to deform the cornea, comprising a monitoring system, with which the deformation of the cornea can be monitored and recorded, it being possible to record sectional images of the undeformed and deformed cornea using the monitoring system, and comprising an analysis device, with which the intraocular pressure can be derived from the sectional images of the cornea,
**characterised in that**
the recorded sectional images of the deformed cornea are corrected relative to a recorded sectional image of the undeformed cornea, the sectional images of the deformed cornea each being corrected by a spatial parallel offset in the direction of an optical axis (12) of the eye or of a device axis of the analysis system, relative to the sectional image of the undeformed cornea, the intraocular pressure being derived taking the correction into account.

## Revendications

1. Procédé d'analyse ophtalmologique pour mesurer une pression intraoculaire à l'intérieur d'un oeil (11) au moyen d'un système d'analyse formé par un dispositif d'actionnement, au moyen duquel une cornée (10) de l'oeil est déformée sans contact, dans lequel, au moyen du dispositif d'actionnement, un jet d'air est appliqué à l'oeil pour déformer la cornée, formé par un système d'observation, au moyen duquel la déformation de la cornée est observée et enregistrée, dans lequel, au moyen du système d'observation, des images en coupe de la cornée non déformée et déformée sont enregistrées, et formé par un dispositif d'analyse, au moyen duquel la pression intraoculaire est dérivée à partir des images en coupe de la cornée, **caractérisé en ce que**
les images en coupe enregistrées de la cornée déformée sont corrigées par rapport à une image en coupe enregistrée de la cornée non déformée, dans lequel les images en coupe de la cornée déformée sont respectivement corrigées d'un déplacement spatial et parallèle dans la direction d'un axe (12) optique de l'oeil ou d'un axe de dispositif du système d'analyse, par rapport à l'image en coupe de la cornée non déformée, dans lequel la pression intraoculaire est dérivée en prenant compte de la correction.

2. Procédé d'analyse selon la revendication 1,
**caractérisé en ce que**
l'image en coupe de la cornée (10) non déformée est utilisée comme un point de référence pour les images en coupe de la cornée déformée.

3. Procédé d'analyse selon la revendication 1 ou 2,
**caractérisé en ce qu'**
une fonction du déplacement est prise en compte.

4. Procédé d'analyse selon l'une quelconque des revendications précédentes,
**caractérisé en ce qu'**
un déplacement d'un globe oculaire est mesuré.

5. Procédé selon l'une quelconque des revendications précédentes, **caractérisé en ce qu'**
un déplacement d'un fond (19) d'oeil est mesuré.

6. Procédé d'analyse selon l'une quelconque des revendications précédentes,
**caractérisé en ce qu'**
un déplacement est dérivé à partir des images en coupe de la cornée (10) déformée et non déformée.

7. Procédé d'analyse selon la revendication 6,
**caractérisé en ce que**
le déplacement est déterminé à partir d'une pluralité de points de référence dans une région de bord des images en coupe, la région étant opposée à l'axe (12) optique ou à l'axe de dispositif.

8. Procédé d'analyse selon l'une quelconque des revendications précédentes,
**caractérisé en ce qu'**
un déplacement maximal est déterminé.

9. Procédé d'analyse selon l'une quelconque des revendications précédentes,
**caractérisé en ce qu'**
une pression de pompage pour générer le jet d'air s'étend sous la forme d'une courbe en cloche (13) par rapport à une durée du jet d'air.

10. Procédé selon la revendication 9,
**caractérisé en ce qu'**
une pression de pompage maximale pour générer le jet d'air est identique lors de mesures précédentes et subséquentes.

11. Procédé d'analyse selon la revendication 9 ou 10,
**caractérisé en ce qu'**
une pression de pompage pour générer le jet d'air est mesurée en atteignant un point d'applanation de la cornée (10).

12. Procédé d'analyse selon l'une quelconque des revendications précédentes,
**caractérisé en ce qu'**
une déformation maximale de la cornée (10) est dérivée à partir des images en coupe de la cornée.

13. Procédé d'analyse selon l'une quelconque des revendications, **caractérisé en ce que**
le système d'observation comprend une caméra et un dispositif d'éclairage dans une disposition Scheimpflug, dans lequel les images en coupe sont enregistrées au moyen de la caméra.

14. Système d'analyse ophtalmologique pour mesurer une pression intraoculaire à l'intérieur d'un oeil (11), comprenant un dispositif d'actionnement, au moyen duquel une cornée (10) de l'oeil peut être déformée sans contact, dans lequel, au moyen du dispositif d'actionnement, un jet d'air peut être appliqué à l'oeil pour déformer la cornée, comprenant un système d'observation, au moyen duquel la déformation de la cornée peut être observée et enregistrée, dans lequel, au moyen du système d'observation, des images en coupe de la cornée non déformée et déformée peuvent être enregistrées, et comprenant un dispositif d'analyse, au moyen duquel la pression intraoculaire peut être dérivée à partir des images en coupe de la cornée,
**caractérisé en ce que**
les images en coupe enregistrées de la cornée déformée sont corrigées par rapport à une image en coupe de la cornée non déformée, dans lequel les images en coupe de la cornée déformée sont respectivement corrigées d'un déplacement spatial et parallèle dans la direction d'un axe (12) optique de l'oeil ou d'un axe de dispositif du système d'analyse, par rapport à l'image en coupe de la cornée non déformée, dans lequel la pression intraoculaire est dérivée en prenant compte de la correction.
